(19) **Europäisches Patentamt European Patent Office Office européen des brevets**

(11) **EP 4 674 869 A1**

(12) **EUROPEAN PATENT APPLICATION**
published in accordance with Art. 153(4) EPC

(43) Date of publication:
**07.01.2026 Bulletin 2026/02**

(21) Application number: **24925129.9**

(22) Date of filing: **15.10.2024**

(51) International Patent Classification (IPC):
**C07K 16/28** (2006.01)   **A61P 35/00** (2006.01)
**A61P 35/04** (2006.01)   **A61K 39/00** (2006.01)

(86) International application number:
**PCT/KR2024/015638**

(87) International publication number:
**WO 2025/244201 (27.11.2025 Gazette 2025/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.05.2024 KR 20240066776**

(71) Applicant: **Immuneoncia Therapeutics, Inc.**
**Yongin-si, Gyeonggi-do 17084 (KR)**

(72) Inventors:
• **KIM, Heung Tae**
**Yongin-si, Gyeonggi-do 17084 (KR)**
• **LEE, Sung Young**
**Yongin-si, Gyeonggi-do 17084 (KR)**
• **KIM, Sung Ho**
**Yongin-si, Gyeonggi-do 17084 (KR)**

(74) Representative: **Ullrich & Naumann PartG mbB**
**Schneidmühlstrasse 21**
**69115 Heidelberg (DE)**

Remarks:
The complete document including Reference Table(s) and the Sequence Listing(s) can be downloaded from the EPO website

(54) **METHOD FOR TREATMENT OF ADVANCED OR METASTATIC SOLID CANCER USING ANTI-CD47 ANTIBODY**

(57)   Provided is a method for treating solid cancer using an anti-CD47 antibody.

[FIG. 16]

EP 4 674 869 A1

## Description

## Technical Field

This application claims priority to Korean Patent Application No. 10-2024-0066776, filed on May 22, 2024, the disclosure of which is incorporated herein by reference.

The present disclosure relates to a method for treating a patient with advanced or metastatic cancer using an anti-CD47 antibody.

Sequence Listing

This application includes a sequence listing that has been submitted electronically in XML format and is incorporated herein by reference in its entirety. A copy of the sequence listing, created on October 15, 2024, is named KC24163.xml and is 10.3 kilobytes in size.

## Background Art

CD47 is a transmembrane protein expressed on the surface of cancer cells and delivers a "don't-eat-me" signal to signal regulatory protein alpha (SIRP$\alpha$), which is a receptor present on phagocytic cells such as macrophages and dendritic cells. Binding of CD47 to SIRP$\alpha$ induces phosphorylation of the immunoreceptor tyrosine-based inhibitory motif in the cytoplasmic domain of SIRP$\alpha$ and recruitment of Src homology phosphatases 1 and 2 (SHP-1 and SHP-2), which leads to inhibition of phagocytic signaling (Oldenborg, 2013). This CD47-mediated inhibitory mechanism is frequently hyperactivated in cells of various cancers, including hematologic malignancies and solid cancers. In various animal models, blockade of CD47/SIRP$\alpha$ interaction has been shown to induce macrophage-mediated phagocytosis of cancer cells and enhance antitumor activity (Tseng D, et al., 2013; Majeti R, et al., 2009). In particular, blockade of CD47 causes dendritic cells to phagocytose cancer cells and subsequently cross-present cancer antigens, thereby inducing antitumor T cell responses (Liu X, et al., 2015). In addition, combination of CD47/SIRP$\alpha$ blockade with chemotherapy or immunotherapy drugs such as those targeting programmed cell death protein 1 (PD-1) and programmed death-ligand 1 (PD-L1) has been shown to enhance anticancer efficacy (Gordon SR, et al., 2017; Liu X, et al., 2018; Lian S, et al., 2019).

Various anticancer agents targeting CD47/SIRP$\alpha$ are currently in preclinical or clinical stages of development. According to the relevant website (ClinicalTrials.gov), twelve drug candidates are currently under development as anticancer agents. Magrolimab (Hu5F9-G4, an anti-CD47 monoclonal antibody) has been reported to be effective when administered as monotherapy in patients with acute myeloid leukemia (AML), myelodysplastic syndrome (MDS), and certain solid cancers, including clear cell ovarian cancer and fallopian tube carcinoma. However, due to recent serious safety concerns regarding magrolimab, discontinuation of clinical studies for hematologic malignancies has been announced, and a hold has also been announced on subject enrollment in clinical trials for solid cancers. Accordingly, there remains a critical need for anticancer agents with safety, tolerability, and efficacy, in particular, for patients with advanced or metastatic cancers for which there are no approved or established therapeutic alternatives.

IMC-002, which is an anti-CD47 antibody, is a human IgG4 antibody containing an S228P mutation that stabilizes the hinge region, thereby preventing Fab exchange. It has been found that IMC-002 targets CD47 and inhibits the interaction between CD47 and SIRP$\alpha$, which enhances macrophage-mediated phagocytosis of cancer cells in vitro and suppresses tumor growth in vivo (xenograft models). IMC-002 does not bind appreciably to red blood cells and thus did not cause hemagglutination in vitro or anemia in in vivo studies. These findings suggest that IMC-002 is safe, well-tolerated, and has the potential to be an effective anticancer agent.

[Prior Art Documents]

[Patent Documents]

(Patent Document 0001) Korean Patent No. 10-2625835

[Non-Patent Documents]

(Non-Patent Document 0001) Kabat et al., Sequences of Proteins of Immunological Interest, 5th Ed., US Dept. of Health and Human Services, PHS, NIH, NIH Publication no. 91-3242, 1991

(Non-Patent Document 0002) Lefranc et al, Dev. Comp. Immunol. 29:185-203; 2005

(Non-Patent Document 0003) Honegger and Pluckthun, J. Mol. Biol. 309(3):657-670; 2001

(Non-Patent Document 0004) Tseng D, et al. 2013, Majeti R, et al. 2009

(Non-Patent Document 0005) Gordon SR, et al. 2017 Liu X, et al. 2018, Lian S, et al. 2019

**Disclosure of Invention**

**Technical Problem**

The object of the present disclosure is to solve all of the above-mentioned problems of the prior art.
An object of the present disclosure is to provide a method of administering an anti-CD47 antibody, a pharmaceutical composition, and a kit, for treating a tumor or inhibiting tumor proliferation in a patient with solid cancer.
The object of the present disclosure is not limited to the above-mentioned objects. The objects of the present disclosure will become clearer from the following description and may be realized by means and combinations thereof as set forth in the claims.

**Solution to Problem**

Representative configurations of the present disclosure to achieve the above-mentioned objects are as follows.
According to an aspect of the present disclosure, there is provided a pharmaceutical composition for treating a tumor or inhibiting tumor proliferation in a patient with advanced or metastatic solid cancer, the composition comprising an effective amount of an anti-CD47 antibody that specifically binds to CD47 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2, wherein the antibody is administered to the patient, without a priming dose, at a dose of about 5 mg/kg to about 30 mg/kg about every 2 weeks to about every 3 weeks.
According to an aspect of the present disclosure, there is provided a method for treating a tumor or inhibiting tumor growth, comprising: (a) selecting a patient with advanced or metastatic solid cancer; and (b) administering to the selected patient an anti-CD47 antibody or a pharmaceutical composition as disclosed herein, without a priming dose, at a dose of about 5 mg/kg to about 30 mg/kg about every 2 weeks to about every 3 weeks, wherein the anti-CD47 antibody specifically binds to CD47 and comprises HCDR1, HCDR2, and HCDR3 contained in the heavy chain variable region of SEQ ID NO: 1, and LCDR1, LCDR2, and LCDR3 contained in the light chain variable region of SEQ ID NO: 2.
According to another aspect of the present disclosure, there is provided a kit for treating a tumor or inhibiting tumor proliferation in a patient with solid cancer, comprising an anti-CD47 antibody together with a manual for using the anti-CD47 antibody, wherein the manual comprises instructions regarding a treatment or inhibition method disclosed herein, comprising administering the antibody to the patient, without a priming dose, at a dose of about 5 mg/kg to about 30 mg/kg about every 2 weeks to about every 3 weeks, and the antibody specifically binds to CD47 and comprises HCDR1, HCDR2, and HCDR3 contained in the heavy chain variable region of SEQ ID NO: 1, and LCDR1, LCDR2, and LCDR3 contained in the light chain variable region of SEQ ID NO: 2.
In some embodiments, the antibody may be a fully human anti-CD47 antibody (for example, an IMC-002 antibody).
In some embodiments, the heavy chain variable region of the antibody may comprise HCDR1 of SEQ ID NO: 5, HCDR2 of SEQ ID NO: 6, and HCDR3 of SEQ ID NO: 7, and the light chain variable region may comprise LCDR1 of SEQ ID NO: 8, LCDR2 of SEQ ID NO: 9, and LCDR3 of SEQ ID NO: 10.
In some embodiments, the heavy chain variable region of the antibody may comprise the amino acid sequence of SEQ ID NO: 1 or an amino acid sequence having at least 95% identity thereto, and the light chain variable region may comprise the amino acid sequence of SEQ ID NO: 2 or an amino acid sequence having at least 95% identity thereto.
In some embodiments, the antibody may not induce a significant level of red blood cell agglutination or anemia in the patient.
In some embodiments, the composition may comprise about 10 to 30 mM sodium phosphate, about 80 to 120 mM sodium chloride, about 50 to 80 mM mannitol, and about 0.01 to 0.1% polysorbate 80, and may have a pH of about 4 to 7.
In some embodiments, the composition may comprise about 20 mM sodium phosphate, about 100 mM sodium chloride, about 65.9 mM mannitol, and 0.05% polysorbate 80, and may have a pH of about 5.5.
In some embodiments, the composition may be in a liquid dosage form for intravenous injection or infusion.
In some embodiments, the solid cancer may be liver cancer (for example, hepatocellular carcinoma), breast cancer (for example, triple-negative breast cancer), or gallbladder cancer.

In some embodiments, the patient may be ineligible for standard therapy or has failed standard therapy.

In some embodiments, the antibody may be administered to the patient about every 2 weeks or about every 3 weeks.

In some embodiments, the antibody may be administered to the patient at a dose of about 20 mg/kg or about 30 mg/kg.

In some embodiments, the patient may have received at least one round of systemic therapy before administration of the anti-CD47 antibody.

In some embodiments, the patient may have stage IV solid cancer with multiple distant metastases or may have locally advanced solid cancer.

In some embodiments, the antibody may be administered in combination with another anticancer agent.

In some embodiments, the antibody may be administered in combination with lenvatinib.

In some embodiments, the antibody may be administered in combination with paclitaxel.

In some embodiments, the antibody may be administered in combination with gemcitabine, carboplatin, or a combination thereof.

In some embodiments, the antibody may be administered concurrently with, sequentially to, in reverse sequence to, or at a therapeutically effective interval from another anticancer agent administered in combination.

In some embodiments, lenvatinib may be administered daily.

In some embodiments, paclitaxel may be administered on day 1 of an administration cycle of the antibody.

In some embodiments, gemcitabine and carboplatin may be administered on days 1 and 8 of an administration cycle of the antibody.

In some embodiments, the treatment or inhibition method may further comprise measuring density of CD47-positive macrophages in a biological sample obtained from the patient before or after administration of the anti-CD47 antibody.

In an aspect of the present disclosure, there is provided a pharmaceutical composition for treating a tumor or inhibiting tumor proliferation in a patient with advanced or metastatic hepatocellular carcinoma, the composition comprising an effective amount of IMC-002 antibody, wherein the antibody is administered to the patient, without a priming dose, at a dose of about 5 mg/kg, about 10 mg/kg, about 20 mg/kg, or about 30 mg/kg about every 2 weeks or about every 3 weeks.

In some embodiments, the pharmaceutical composition for treating a tumor or inhibiting tumor proliferation in a patient with advanced or metastatic hepatocellular carcinoma may be administered to the patient in combination with lenvatinib.

In an aspect of the present disclosure, there is provided a pharmaceutical composition for treating a tumor or inhibiting tumor proliferation in a patient with advanced or metastatic gallbladder cancer, the composition comprising an effective amount of IMC-002 antibody, wherein the antibody is administered to the patient, without a priming dose, at a dose of about 5 mg/kg, about 10 mg/kg, about 20 mg/kg, or about 30 mg/kg about every 2 weeks or about every 3 weeks.

In an aspect of the present disclosure, there is provided a pharmaceutical composition for treating a tumor or inhibiting tumor proliferation in a patient with advanced or metastatic breast cancer, the composition comprising an effective amount of IMC-002 antibody, wherein the antibody is administered to the patient, without a priming dose, at a dose of about 5 mg/kg, about 10 mg/kg, about 20 mg/kg, or about 30 mg/kg about every 2 weeks or about every 3 weeks.

In some embodiments, the pharmaceutical composition for treating a tumor or inhibiting tumor proliferation in a patient with advanced or metastatic breast cancer may be administered to the patient in combination with paclitaxel or a combination of gemcitabine and carboplatin.

### Effects of Invention

The anti-CD47 antibody IMC-002 induces macrophage-mediated phagocytosis of cancer cells and strongly inhibits tumor growth in patients with advanced or metastatic cancers. In patients with advanced or metastatic solid cancers who had failed standard therapy, administration of IMC-002 at a dose of about 5 mg/kg to about 30 mg/kg about every 2 weeks to about every 3 weeks without a priming dose demonstrated an excellent safety profile and therapeutic efficacy.

### Brief Description of Drawings

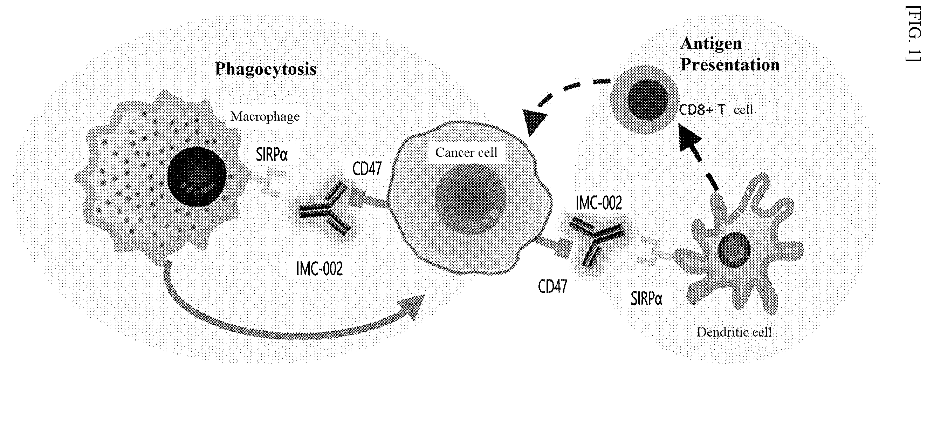

FIG. 1 illustrates the mechanism of action of an anti-CD47 antibody (IMC-002) according to an embodiment of the present disclosure.

FIG. 2 illustrates a schematic diagram for the dose-escalation study and the expansion study according to the clinical trial design of an embodiment of the present disclosure.

FIG. 3 illustrates clinical trial results according to an embodiment of the present disclosure, showing changes in hemoglobin levels depending on different dose levels of IMC-002 in the dose-escalation study.

FIG. 4 illustrates clinical trial results according to an embodiment of the present disclosure, showing changes in neutrophil counts depending on different dose levels of IMC-002 in the dose-escalation study.

FIG. 5 illustrates clinical trial results according to an embodiment of the present disclosure, showing changes in platelet counts depending on different dose levels of IMC-002 in the dose-escalation study.

FIG. 6 illustrates clinical trial results according to an embodiment of the present disclosure, showing a swimmer plot of tumor responses over time in the dose-escalation study. In the swimmer plot, the symbol • indicates stable disease (SD), the symbol + indicates progressive disease (PD), and the symbol → indicates ongoing treatment.

FIG. 7 illustrates clinical trial results according to an embodiment of the present disclosure, showing percent changes in tumor burden of target lesions in the dose-escalation study.

FIGS. 8A and 8B illustrate clinical trial results according to an embodiment of the present disclosure, showing densities of CD47-positive and CD47-negative macrophages in patients who experienced clinical benefit (CBR) and those who did not (Non-CBR).

FIG. 9 illustrates clinical trial results according to an embodiment of the present disclosure, showing a simulated pharmacokinetic (PK) profile of IMC-002 following intravenous infusion every 3 weeks.

FIG. 10 illustrates clinical trial results according to an embodiment of the present disclosure, showing a comparison of pharmacokinetic (PK) profiles depending on different dose levels.

FIG. 11 illustrates clinical trial results according to an embodiment of the present disclosure, showing serum PK concentrations following initial administration.

FIG. 12 illustrates clinical trial results according to an embodiment of the present disclosure, showing changes in hemoglobin levels depending on different dose levels of IMC-002 in the expansion study.

FIG. 13 illustrates clinical trial results according to an embodiment of the present disclosure, showing changes in neutrophil counts depending on different dose levels of IMC-002 in the expansion study.

FIG. 14 illustrates clinical trial results according to an embodiment of the present disclosure, showing changes in platelet counts depending on different dose levels of IMC-002 in the expansion study.

FIG. 15 illustrates clinical trial results according to an embodiment of the present disclosure, showing a swimmer plot of tumor responses over time in the expansion study. In the swimmer plot, the symbol ▲ indicates partial response (PR), the symbol • indicates stable disease (SD), the symbol + indicates progressive disease (PD), and the symbol→ indicates ongoing treatment.

FIG. 16 illustrates clinical trial results according to an embodiment of the present disclosure, showing percent changes in tumor burden of target lesions in the expansion study.

## Mode for Carrying out Invention

The detailed description of the present disclosure described below will be described with reference to specific drawings (if any) regarding specific embodiments in which the present disclosure may be practiced, and the present disclosure is not limited thereto, but is limited only by the appended claims with respect to any scope identical to or equivalent to that described in the claims. It should be understood that the various embodiments/examples of the present disclosure are different from each other but do not need to be mutually exclusive. For example, certain shapes, structures, and features described herein may be altered from one embodiment/example to another, or may be realized in a combination of a plurality of embodiments/examples, without departing from the technical ideas and scope of the present disclosure. Technical and academic terms used in this specification, unless otherwise defined, have the same meanings as those commonly used in the field to which the present disclosure belongs. In case of a conflict, the specification that contains the definition shall control. The definitions of terms set forth in this specification shall apply for the purpose of interpreting this specification, and terms expressed in the singular form shall be construed to refer also to the plural form (that is, at least one) unless the context makes it inappropriate, and vice versa.

**Definitions**

As used herein, the term "about" refers to a typical range of errors for each value known to those skilled in the art. In addition, unless otherwise specified, all numbers, values and/or expressions, which are used herein to describe ingredients, conditions, compositions, amounts, and the like, are to be understood as being modified by the term "about," as these numerical values are inherently approximations that reflect, among other things, various uncertainties of measurement occurring in obtaining such values. In an example, the term may include cases where a given numerical value $\pm 10\%$ or less, $\pm 9\%$ or less, $\pm 8\%$ or less, $\pm 7\%$ or less, $\pm 6\%$ or less, $\pm 5\%$ or less, $\pm 4\%$ or less, $\pm 3\%$ or less, $\pm 2\%$ or less, $\pm 1\%$ or less, or $\pm 0.5\%$ or less.

The term "cancer" refers to a variety of diseases characterized by uncontrolled growth of abnormal cells in the body. The term "cancer" or "cancerous tissue" may include a tumor.

The term "advanced," as used in relation to cancer, refers to cancer that cannot be completely removed or controlled by surgery and is locally advanced. "Advanced cancer" refers to cancer that has progressed to at least stage IIIB.

The term "metastatic," as used in relation to cancer, refers to a state of cancer where cancer cells break away from where they first formed, travel through, for example, the blood or lymphatic system, and form tumors (metastatic tumors) in other parts of the body. "Metastatic cancer" refers to cancer that has progressed to at least stage IV.

The term "standard therapy" refers to a treatment modality generally accepted by medical professionals as appropriate for treatment of specific cancer, preferably a specific solid tumor. The "standard therapy" may be the same or different depending on different tumor types. The "standard therapy" may include first-line or second-line treatment options, such as surgery, radiotherapy, chemotherapy, and cancer immunotherapy. The "standard therapy" may be approved by various regulatory authorities, such as the U.S. Food and Drug Administration (FDA).

The term "antibody" includes monoclonal antibodies (including full-length antibodies) of any isotype, such as IgG, IgM, IgA, IgD, and IgE, polyclonal antibodies, multispecific antibodies (for example, bispecific antibodies), antibody fusions (for example, a fusion of an antibody with a (poly)peptide or a fusion of an antibody with a compound), and antibody fragments (including antigen-binding fragments). As used herein, the prefix "anti-," when in conjunction with an antigen, indicates that the given antibody is reactive with the given antigen. An antibody reactive with a specific antigen may be produced, by way of non-limiting example, through synthetic and/or recombinant methods such as selection from libraries of recombinant antibodies in phage or similar vectors, or by immunizing an animal with the antigen or an antigen-encoding nucleic acid. A typical IgG antibody is comprised of two identical heavy chains and two identical light chains which are linked by disulfide bonds. Each heavy and light chain contains a constant region and a variable region. Each of the heavy chain variable region (HVR) and the light chain variable region (LVR) contains three segments, called "complementarity determining regions" ("CDRs") or "hypervariable regions," which are primarily responsible for binding an epitope of an antigen. They are usually referred to as CDR1, CDR2, and CDR3, numbered sequentially from the N-terminus. The more highly conserved portions of the variable regions outside the CDRs are called "framework regions" ("FRs"). As used herein, the antibody may be, for example, an animal antibody, a chimeric antibody, a humanized antibody, or a human antibody.

The term "monoclonal antibody" or "mAb" refers to a population of antibodies that are substantially homogeneous, wherein the individual antibody molecules comprising the population are identical in amino acid sequence, except for possible naturally occurring mutations that may be present in minor amounts. In contrast, conventional (polyclonal) antibody preparations typically comprise a number of different antibodies having different amino acid sequences in their variable domains, in particular, their CDRs, that are specific for different epitopes. The modifier "monoclonal" indicates that the antibody is obtained from a population of antibodies that are substantially homogeneous in character and should not be construed as requiring antibody production by any particular method. For example, the monoclonal antibody used according to the treatment methods, pharmaceutical compositions, or disclosed uses may be produced by the hybridoma method first described in Kohler et al. (1975), Nature 256: 495, or a recombinant deoxyribonucleic acid (DNA) method. "Monoclonal antibodies" may also be isolated from phage antibody libraries using techniques described, for example, in Clackson et al. (1991) Nature 352: 624-628 and Marks et al. (1991) J. Mol. Biol. 222: 581-597. See also Presta (2005) J. Allergy Clin. Immunol. 116: 731.

The term "priming" refers to an initial antigenic stimulation administered prior to the initiation of therapy. "Priming" is used to activate the immune system or to maximize effects of therapy. For example, in certain immunotherapies, a "priming" dose may activate T cells so that subsequent treatments work more effectively. "Priming" refers to a primary antigenic stimulation that induces an immune response to a specific antigen and establishes recall of a heightened immune response to the specific antigen upon subsequent reimmunization of the same antigen.

The term "pharmaceutical composition" or "pharmaceutical formulation" refers to a combination of an active ingredient with an inert or active carrier and/or excipient.

The term "effective amount" refers to an amount of a compound (including macromolecules such as antibodies) or composition (for example, a compound or composition of the present disclosure) that is sufficient to effect beneficial or desired results. An effective amount may be administered in one or more administrations, applications, or dosages, and is not intended to be limited to any particular formulation or route of administration.

The term "patient" or "subject" refers to a single entity to whom a therapy or treatment method is indicated, who is participating in a clinical trial or epidemiological study, or who is used as a control. The term includes human subjects as well as veterinary patients that are mammals, such as cattle, horses, dogs, and cats.

The term "administration" refers to the physical introduction of a therapeutic agent into a subject using any of a variety of methods and delivery systems known to those skilled in the art. Exemplary routes of administration include oral, intravenous, intramuscular, subcutaneous, intraperitoneal, spinal, or other parenteral routes. Parenteral routes of administration may include, for example, injection or infusion (for example, intravenous infusion). When administered orally, the active ingredient may be formulated with pharmaceutically acceptable excipients into pills, capsules, sachets, tablets, or the like. As used herein, the term "parenteral administration" generally refers to administration other than enteral or topical routes and typically involves injection, including, without limitation, intravenous, intramuscular, intraarterial, intrathecal, intralymphatic, intralesional, intracapsular, intraorbital, intracardiac, intradermal, intraperitoneal, transtracheal, subcutaneous, subepidermal, intraarticular, subcapsular, subarachnoid, intraspinal, epidural, and intrasternal injection or infusion, as well as in vivo electroporation. The therapeutic agent may be administered either parenterally or orally. Other parenteral routes of administration include topical, transdermal, or mucosal routes, such as intranasal, vaginal, rectal, sublingual, or local administration. Administration may be performed, for example, once, multiple times, and/or over an extended period of one or more days.

The term "combination administration" refers to the administration of two or more therapeutic agents. These respective types of therapeutic agents may be administered to a subject concurrently, sequentially, or individually. Concurrent administration means administering the respective types of therapeutic agents at the same time via the same or different administration methods, and sequential administration means administering the respective types of therapeutic agents separately and consecutively at a defined dosing interval, wherein the time required for the dosing interval is minimized as much as possible. Individual administration means administering the respective types of therapeutic agents at a defined time interval. Such a mode of administration may be appropriately selected by a person skilled in the art in consideration of the therapeutic efficacy and adverse effects in the patient. The number of administrations may also be appropriately selected, along with the dosage, in consideration of the patient's disease condition and therapeutic efficacy. For example, administration may be performed once or multiple times, and may be carried out over one or more extended periods.

The term "biological sample" encompasses a clinical sample, and also includes tissue obtained by surgical resection, tissue obtained by biopsy, cells in culture, cell supernatants, cell lysates, tissue samples, organs, bone marrow, blood, plasma, serum, and the like. A "biological sample" includes a sample comprising target cells or normal control cells or suspected of comprising such cells or biological fluids derived therefrom (for example, cancerous cells, infected cells, and the like), for example, a sample comprising polynucleotides and/or polypeptides which is obtained from such cells (for example, cell lysates or other cell extracts comprising polynucleotides and/or polypeptides). A biological sample comprising an inflicted cell from a patient can also include non-inflicted cells.

The terms "phagocytic cells" and "phagocytes" are used interchangeably herein to refer to cells capable of phagocytosis. There are three main categories of phagocytes: macrophages, mononuclear cells (histiocytes and monocytes); polymorphonuclear leukocytes (neutrophils) and dendritic cells.

**Method of Treatment**

In an aspect of the present disclosure, there is provided a method of treating a patient with an effective amount of an anti-CD47 agent. The present disclosure is based in part on clinical findings that in a case where an anti-CD47 antibody (in particular, IMC-002) is administered to a patient with advanced or metastatic solid cancer at a specific dosage and schedule, both safety and efficacy can be achieved without a priming dose.

In some embodiments, the cancer may be advanced or metastatic solid cancer. Specifically, the solid cancer may be one or more selected from the group consisting of lung cancer, pancreatic cancer, colon cancer, colorectal cancer, gallbladder cancer, biliary tract cancer, gastric cancer, liver cancer, brain cancer, breast cancer, thyroid cancer, bladder cancer, esophageal cancer, ovarian cancer, melanoma, head and neck cancer, skin cancer, prostate cancer, hepatocellular carcinoma, fibrosarcoma, and cervical cancer. More specifically, the solid cancer may be liver cancer (for example, hepatocellular carcinoma), breast cancer (for example, triple-negative breast cancer), or gallbladder cancer.

In some embodiments, the patient may be ineligible for standard therapy or has failed standard therapy. Specifically, the standard therapy may refer to a treatment modality generally accepted by medical professionals as appropriate for treatment of specific cancer, preferably a specific solid tumor. Specifically, the standard therapy may include first-line or second-line treatment options, such as surgery, radiotherapy, chemotherapy, cancer immunotherapy, or a combination thereof. Failure of standard therapy may mean that relapse/refractory disease/disease progression has been confirmed following the standard therapy.

In some embodiments, the patient may have stage IV solid cancer with multiple distant metastases or have locally advanced solid cancer. Such patients may include, for example, those having histologically or cytologically confirmed metastatic or locally advanced liver cancer (for example, hepatocellular carcinoma), breast cancer (for example, triple-negative breast cancer), or gallbladder cancer.

In some embodiments, the method of the present disclosure does not comprise a step of admininstering a priming dose of an anti-CD47 agent. Specifically, absence of the priming dose administration step means that a preparatory step to activate the immune system in the early stages of cancer therapy is omitted. Omission of a priming dose can simplify the treatment process and may be particularly advantageous for elderly patients or those with other health issues. In addition, omission of a priming dose may reduce adverse effects associated with cancer therapy.

In some embodiments, the anti-CD47 agent of the present disclosure may be an anti-CD47 antibody, specifically a fully human anti-CD47 monoclonal antibody. The fully human antibody of the present disclosure is an antibody that is produced by a human and/or has an amino acid sequence corresponding to that of an antibody produced using any technique for producing human antibodies.

In some embodiments, the anti-CD47 antibody of the present disclosure is an IgG4 antibody. Specifically, the anti-CD47 antibody is an antibody of the IgG4 kappa isotype. More specifically, the anti-CD47 antibody is comprised of a glycosylated tetramer having two identical gamma heavy chains and two identical kappa light chains, which are linked by disulfide bonds.

In some embodiments, the anti-CD47 antibody of the present disclosure contains an S228P mutation in the hinge region, thereby preventing Fab exchange. The S228P mutation reduces Fab-arm exchange.

In some embodiments, the anti-CD47 antibody may comprise HCDR1, HCDR2, and HCDR3 contained in the heavy chain variable region of SEQ ID NO: 1, and LCDR1, LCDR2, and LCDR3 contained in the light chain variable region of SEQ ID NO: 2.

In some embodiments, the heavy chain variable region of the anti-CD47 antibody may comprise or consist of the amino acid sequence of SEQ ID NO: 1, or an amino acid sequence having at least 95%, at least 96%, at least 97%, at least 98%, or at least 99% identity to the sequence set forth in SEQ ID NO: 1.

In some embodiments, the light chain variable region of the anti-CD47 antibody may comprise or consist of the amino acid sequence of SEQ ID NO: 2, or an amino acid sequence having at least 95%, at least 96%, at least 97%, at least 98%, or at least 99% identity to the sequence set forth in SEQ ID NO: 2.

In some embodiments, the anti-CD47 antibody may comprise the heavy chain variable region of SEQ ID NO: 1 and/or the light chain variable region of SEQ ID NO: 2.

In some embodiments, the anti-CD47 antibody may comprise the heavy chain of SEQ ID NO: 3 and/or the light chain of SEQ ID NO: 4.

In some embodiments, the anti-CD47 antibody may be an antibody designated as IMC-002. IMC-002 is a fully human anti-CD47 antibody of IgG4 comprising a heavy chain variable domain comprising the amino acid sequence set forth in SEQ ID NO: 1 and a light chain variable domain comprising the amino acid sequence set forth in SEQ ID NO: 2. IMC-002 comprises an S228P mutation in the hinge region. For the amino acid sequence information of IMC-002, see Table 1 below.

[Table 1]

| Category | Sequence | SEQ ID NO |
|---|---|---|
| Heavy chain variable domain | QVQLVQSGAEVKKPGASVKVSCKAS**GYTFTSY**YMHWVRQAPGQGL EWMGII**NPSGGS**TSYAQKFQGRVTMTRDTSTSTVYMELSSLRSEDTA VYYCAR**STLWVSEFDY**WGQGTLVTVSS | 1 |
| Light chain variable domain | QSALTQPASVSGSPGQSISISC**TGTSSDVGGHNYVS**WYQQHPGKAPK LMIY**DVRNRPS**GVSNRFSGSKSGSTASLTISGLQTEDEADYYC**NSYTG SRTYV**FGSGTKLTVL | 2 |

(continued)

| Category | Sequence | SEQ ID NO |
|---|---|---|
| Heavy chain | QVQLVQSGAEVKKPGASVKVSCKASGYTFTSYYMHWVRQAPGQGL EWMGIINPSGGSTSYAQKFQGRVTMTRDTSTSTVYMELSSLRSEDTAV YYCARSTLWVSEFDYWGQGTLVTVSSASTKGPSVFPLAPCSRSTSEST AALGCLVKDYFPEPVTVSWNSGALTSGVHTFPAVLQSSGGLYSLSSVVT VPSSSLGTKTYTCNVDHKPSNTKVDKRVESKYGPPCPPCPAPEFLGGP SVFLFPPKPKDTLMISRTPEVTCVVVDVSQEDPEVQFNWYVDGVEVH NAKTKPREEQFNSTYRVVSVLTVLHQDWLNGKEYKCKVSNKGLPSSI EKTISKAKGQPREPQVYTLPPSQEEMTKNQVSLTCLVKGFYPSDIAVE WESNGQPENNYKTTPPVLDSDGSFFLYSRLTVDKSRWQEGNVFSCSV MHEALHNHYTQKSLSLSLGK | 3 |
| Light chain | QSALTQPASVSGSPGQSISISCTGTSSDVGGHNYVSWYQQHPGKAPKL MIYDVRNRPSGVSNRFSGSKSGSTASLTISGLQTEDEADYYCNSYTGS RTYVFGSGTKLTVLGQPKAAPSVTLFPPSSEELQANKATLVCLISDFYP GAVTVAWKADSSPVKAGVETTTPSKQSNNKYAASSYLSLTPEQWKSH RSYSCQVTHEGSTVEKTVAPTECS | 4 |
| Heavy chain variable domain CDR1 | GYTFTSY | 5 |
| Heavy chain variable domain CDR2 | NPSGGS | 6 |
| Heavy chain variable domain CDR3 | STLWVSEFDY | 7 |
| Light chain variable domain CDR1 | TGTSSDVGGHNYVS | 8 |
| Light chain variable domain CDR2 | DVRNRPS | 9 |
| Light chain variable domain CDR | NSYTGSRTYV | 10 |

IMC-002 is the antibody clone C47B10-H3-D4 disclosed in Korean Patent No. 10-2625835 (corresponding to International Patent Application No. PCT/US2016/020980), which was selected from among various anti-CD47 antibodies disclosed therein based on its low red blood cell agglutination in an in vitro red blood cell agglutination assay. The entire disclosure of the above document is incorporated herein by reference.

In some embodiments, the dosage of the anti-CD47 antibody may vary depending on factors such as the age and weight of the subject to be administered, the target disease, the pathological condition, and the route of administration. In a case where the anti-CD47 antibody is used for treating solid cancer or inhibiting proliferation thereof, it may be administered as a single dose or in multiple doses. Depending on severity of the pathological condition, the frequency and duration of treatment may be adjusted.

In some embodiments, the method may comprise administering the anti-CD47 antibody to the subject's tumor in a therapeutically effective amount one or more times, for example, once, twice, three times, four times, five times, six times, seven times, eight times, nine times, or ten or more times. For example, a therapeutic dosing regimen may comprise administering the anti-CD47 antibody one or more times at intervals of about 1 day, about 2 days, about 3 days, about 4 days, about 5 days, about 6 days, about 1 week, about 2 weeks, about 3 weeks, or about 4 weeks. In some embodiments, the anti-CD47 antibody may be administered about every 2 weeks to 3 weeks. In some embodiments, the anti-CD47 antibody may be administered about every 2 or 3 weeks.

In some embodiments, each dose of the anti-CD47 antibody may be about 5 to about 30 mg/kg. For example, each dose of the anti-CD47 antibody may be about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, or about 40 mg/kg. In some embodiments, each dose of the anti-CD47 antibody may be about 20 to about 30 mg/kg. In some embodiments, each dose of the anti-CD47 antibody is about 20 mg/kg or about 30 mg/kg.

In some embodiments, the minimum effective concentration of the anti-CD47 antibody for treatment of solid cancer may be about 20 to about 30 $\mu$g/mL. In some embodiments, the minimum effective concentration of the anti-CD47 antibody for treatment of solid cancer may be about 24 $\mu$g/mL. The minimum effective concentration refers to the lowest concentration of a drug that must be maintained in the serum for treatment of solid cancer. In some embodiments, upon administration of the pharmaceutical composition of the present disclosure, the drug concentration in the serum may be equal to or greater than about 20 to about 30 $\mu$g/mL, which corresponds to the minimum effective concentration. In some embodiments, upon administration of the pharmaceutical composition of the present disclosure, the drug concentration in the serum may be equal to or greater than about 24 $\mu$g/mL, which corresponds to the minimum effective concentration.

**Pharmaceutical Composition**

In another aspect of the present disclosure, the anti-CD47 agent disclosed herein may be provided as a pharmaceutical composition suitable for therapeutic use, for example, for treatment of a human. In some embodiments, the anti-CD47 antibody disclosed herein may be formulated with one or more carriers and/or excipients to provide a pharmaceutical composition. In some embodiments, there is provided a pharmaceutical composition comprising the anti-CD47 antibody disclosed herein and one or more carriers and/or excipients. Specifically, a therapeutic formulation comprising the anti-CD47 antibody may be prepared for storage by mixing the anti-CD47 antibody, which has a desired level of purity, with optional physiologically acceptable carriers, excipients, or stabilizers, in the form of a lyophilized preparation or an aqueous solution. For the carriers, excipients, and stabilizers, reference may be made to Remington's Pharmaceutical Sciences, 16th edition, Osol, A. Ed. (1980). The preferred form of the composition may depend on the intended mode of administration and therapeutic application. In some embodiments, the composition may further comprise a pharmaceutically acceptable, non-toxic carrier or diluent, defined as a vehicle typically used in formulating pharmaceutical compositions for administration to animals or humans, depending on the desired formulation. The diluent is selected so as not to affect the biological activity of the composition. Examples of the diluents include distilled water, physiologic phosphate-buffered saline, Ringer's solution, dextrose solution, and Hank's solution. The pharmaceutical composition or formulation may also comprise other carriers, adjuvants, or non-toxic, nontherapeutic, and non-immunogenic stabilizers, and the like.

Typically, the pharmaceutical composition is prepared as an injectable, either as a liquid solution or suspension; and a solid form suitable for solution in, or suspension in, a liquid vehicle prior to injection may also be prepared. The preparation may be emulsified or encapsulated in liposomes or microparticles, such as polylactide, polyglycolide, or copolymer for enhanced adjuvant effect, as discussed above. The agents of the present disclosure may also be administered in the form of a depot injection or implant preparation which can be formulated in such a manner as to permit a sustained or pulsatile release of the active ingredient. The pharmaceutical composition is generally formulated as a sterile, substantially isotonic and in full compliance with all Good Manufacturing Practice (GMP) regulations of the U.S. Food and Drug Administration.

In some embodiments, the pharmaceutical composition may be an aqueous composition.

In some embodiments, the pharmaceutical composition may comprise the anti-CD47 antibody of the present disclosure together with about 10 to 30 mM sodium phosphate as a phosphate buffer, about 80 to 120 mM sodium chloride, about 50 to 80 mM mannitol, and about 0.01% to 0.1% polysorbate 80.

In some embodiments, the pharmaceutical composition may comprise the anti-CD47 antibody of the present disclosure together with about 20 mM sodium phosphate, about 100 mM sodium chloride, about 65.9 mM mannitol, and about 0.05% polysorbate 80.

In some embodiments, the pharmaceutical composition may have a pH of about 4 to 7, specifically about 5 to 6, and more specifically about 5.5.

In some embodiments, the pharmaceutical composition may comprise the anti-CD47 antibody of the present disclosure at a concentration of about 40 to 60 mg/mL, specifically at about 50 mg/mL.

In some embodiments, the anti-CD47 antibody of the present disclosure may be provided as a liquid formulation in a 10 mL sterile glass vial.

The anti-CD47 agent may be administered by any suitable route and mode, including oral or parenteral administration. In some embodiments, the anti-CD47 agent is administered parenterally. Parenteral administration refers to administration other than enteral and topical routes and typically involves injection, including epidermal, intravenous, intramuscular, intraarterial, intrathecal, intracapsular, intraorbital, intracardiac, intradermal, intraperitoneal, intratendinous, transtracheal, subcutaneous, subepidermal, intraarticular, subcapsular, subarachnoid, intraspinal, intracranial, intrapleural, epidural, and intrasternal injection or infusion. In some embodiments, the route of administration of the anti-CD47 agent is intravenous injection or infusion. In some embodiments, the route of administration of the anti-CD47 agent is intravenous infusion.

**Combination Administration**

In an aspect of the present disclosure, the anti-CD47 antibody of the present disclosure may be administered in combination with another anticancer agent. For example, the other anticancer agent may include, but is not limited to, one or more of the following: chemotherapeutic agents (for example, carboplatin, gemcitabine, paclitaxel, or combinations thereof); and tyrosine kinase inhibitors (for example, lenvatinib, regorafenib, cabozantinib, or combinations thereof).

In some embodiments, the anti-CD47 antibody of the present disclosure may be administered in combination with a tyrosine kinase inhibitor, preferably lenvatinib, regorafenib, cabozantinib, or a combination thereof, and more preferably in combination with lenvatinib.

In some embodiments, the anti-CD47 antibody of the present disclosure may be administered in combination with a chemotherapeutic agent, preferably paclitaxel, carboplatin, gemcitabine, or a combination thereof, and more preferably in combination with paclitaxel or with carboplatin and gemcitabine.

In some embodiments, the anti-CD47 antibody may be administered in combination with lenvatinib to a patient with hepatocellular carcinoma. In some embodiments, during the combination administration, lenvatinib may be administered once a day. In some embodiments, during the combination administration, lenvatinib may be administered once a day at a dose of 4 to 8 mg (for example, 8 mg) for patients weighing less than 60 kg, or at a dose of 4 to 12 mg (for example, 12 mg) for patients weighing 60 kg or more.

In some embodiments, the anti-CD47 antibody may be administered in combination with paclitaxel to a patient with triple-negative breast cancer. In some embodiments, during the combination administration, paclitaxel may be administered on days 1 to 3 of each cycle, preferably on day 1 of each cycle. In some embodiments, during the combination administration, paclitaxel may be administered by intravenous (IV) infusion at a dose of 100 to 250 mg/m$^2$ on days 1 to 3 of each cycle. For example, paclitaxel may be administered by IV infusion at a dose of 175 mg/m$^2$ on day 1 of each cycle.

In some embodiments, the anti-CD47 antibody may be administered in combination with gemcitabine and carboplatin to a patient with triple-negative breast cancer. In some embodiments, during the combination administration, gemcitabine and carboplatin may each be administered once to three times per each cycle, preferably twice per each cycle. In some embodiments, during the combination administration, gemcitabine and carboplatin may each be administered on days 1 to 3 and days 6 to 10 of each cycle. In some embodiments, the during combination administration, gemcitabine and carboplatin may each be administered on days 1 and 8 of each cycle. In some embodiments, gemcitabine may be administered by IV infusion at a dose of 800 to 1,200 mg/m$^2$ and carboplatin may be administered by IV infusion at an AUC of 1.5 to 2.5, on days 1 to 3 and days 6 to 10 of each cycle. For example, gemcitabine may be administered by IV infusion at a dose of 1,000 mg/m$^2$ and carboplatin may be administered by IV infusion at an AUC of 2, on days 1 and 8 of each cycle.

**Kit**

In yet another aspect of the present disclosure, there is provided a kit for using the anti-CD47 pharmaceutical composition disclosed herein. The kit comprises the anti-CD47 agent disclosed herein. In some embodiments, the kit comprises two or more anti-CD47 agents. In some embodiments, the anti-CD47 agent is provided in a dosage form (for example, a therapeutically effective dosage form). In some embodiments, the anti-CD47 agent is provided in two or more different dosage forms (for example, two or more different therapeutically effective dosage forms). In some embodiments, the anti-CD47 agent may be provided in a liquid or solid form in any convenient packaging (for example, stick pack, dose pack, and the like).

In some embodiments, the kit may further comprise instructions for carrying out the method. The instructions for carrying out the method are the treatemnt or inhibition methods disclosed herein. The instructions may be present in the kit in various forms, one or more of which may be present in the kit. One form in which such instructions may be present is as prinited information on a suitable medium or substrate, such as a piece or pieces of paper on which the information is printed, in the packaging of the kit, in a package insert, and the like. Another form of such instructions is a computer-readable medium, such as a diskette, compact disc (CD), flash drive, and the like, on which the information has been recorded. Yet another form of such instructions that may be present is a website address that may be used via the internet to access the information at a removed site.

All patents and publications cited herein are incorporated by reference in their entirety.

The present disclosure will now be described in further detail with reference to the following examples. However, the following examples are provided for illustrative purposes only and are not intended to limit the scope of the present disclosure.

<u>Examples</u>

**Example 1: Phase 1 dose-escalation and expansion study evaluating the safety, tolerability, pharmacokinetics, and clinical activity of IMC-002 in patients with advanced or metastatic solid cancers**

This example describes a dose-escalation and expansion cohort study of the anti-CD47 antibody IMC-002, conducted in patients with metastatic or locally advanced solid cancers who were ineligible for standard therapy or whose cancers were histologically or cytologically confirmed after standard therapy.

## 1.1. Background and Rationale

The pharmacological data of IMC-002 demonstrated its potential as an anticancer agent, and the nonclinical safety, pharmacokinetic, and pharmacodynamic data thereof were used to determine a starting dose for a clinical trial. In addition, the results from the completed clinical trial in patients with metastatic or locally advanced solid cancers suggested that IMC-002 was safe and well-tolerated when administered weekly or every other week.

CD47 is a transmembrane glycoprotein that is ubiquitously expressed on the surface of various cell types and overexpressed in a wide range of cancer cells. IMC-002 is a fully human IgG4 antibody targeting CD47, and lacks Fc effector functions including complementdependent cytotoxicity (CDC) and antibody-dependent cell-mediated cytotoxicity (ADCC). IMC-002 bound to various cancer cells such as MDA-MB-231 ($EC_{50}$ = 5.73 $\mu$g/mL) and Raji ($EC_{50}$ = 4.77 $\mu$g/mL); however, unlike other CD47-blocking agents, it did not bind to red blood cells even at concentrations up to 300 $\mu$g/mL, and therefore did not induce red blood cell agglutination.

An in vitro phagocytosis assay was used to evaluate whether IMC-002 could induce antibody-dependent cellular phagocytosis (ADCP). As a single agent, IMC-002 induced phagocytosis in a dose-dependent manner against various cancer cell lines derived not only from solid cancers but also from hematologic tumors.

In vivo efficacy of IMC-002 was evaluated in a mouse xenograft model. The MDA-MB-231 xenograft model was used to test the efficacy of IMC-002. As a result, administration of IMC-002 at 3 mg/kg or higher at one-week intervals significantly inhibited tumor growth and prolonged survival. In addition, the in vivo efficacy of IMC-002 was also demonstrated in the MDA-MB-231 orthotopic xenograft model when administered three times per week at 10 mg/kg. No drug-related body weight loss or abnormal clinical signs were observed in any of the test models. Pharmacokinetic (PK) properties of IMC-002 were evaluated in mice and monkeys. IMC-002 was injected intravenously into C57BL/6 mice and cynomolgus monkeys at doses ranging from 0.4 to 10.0 mg/kg. Here, IMC-002 exhibited overall dose-proportional systemic exposure. The volume of distribution at steady state (Vdss) was 75.1 to 83.8 mL/kg in mice and 137.9 to 200.3 mL/kg in monkeys. The total body clearance (CL) was 0.2 to 0.3 mL/hr/kg in mice and 0.7 to 1.1 mL/hr/kg in monkeys. The serum concentrations decreased while exhibiting multiphasic pharmacokinetics, with a terminal elimination half-life ($t_{1/2}$) of 224.9 to 240.9 hours in mice and 114.8 to 171.6 hours in monkeys.

In a 15-day preliminary toxicity study conducted in monkeys, IMC-002 was intravenously administered at a dose of 100 mg/kg three times at one-week intervals. As a result, IMC-002 demonstrated good tolerability and showed minimal decreases in red blood cell and platelet counts. In a 4-week repeated-dose toxicity study conducted in monkeys, IMC-002 was administered intravenously at doses of 10, 30, and 100 mg/kg at one-week intervals for 4 weeks, followed by a 4-week recovery period to assess reversibility. No abnormalities were observed in clinical signs, general behavior and neurofunctional activity, body weight, food consumption, ophthalmic examination, electrocardiogram, respiratory rate, blood pressure, urinalysis, clinical chemistry, cytokine analysis, necropsy findings, organ weights, and histopathology. Investigational drug-related changes were observed in hematology and peripheral blood immunophenotyping; however, such changes were considered to be of low toxicological significance, with individual values being within or slightly outside the reference ranges. No corresponding changes were observed in clinical pathology or histopathological analyses. As a result, the no observed adverse effect level (NOAEL) of IMC-002 was determined to be 100 mg/kg.

An in vivo safety pharmacology test was conducted to evaluate adverse effects of IMC-002 on the central nervous, respiratory, and cardiovascular systems during the 4-week toxicity study in monkeys. No investigational drug-related adverse changes were observed in the nervous, respiratory, or cardiovascular system.

## 1.2. IMC-002 Agent

The active pharmaceutical ingredient is IMC-002, which is a fully human anti-CD47 monoclonal antibody of the IgG4 kappa isotype. IMC-002 is comprised of a glycosylated tetramer having two identical 446-amino acid gamma heavy chains and two identical 216-amino acid kappa light chains, which are linked by disulfide bonds. The IMC-002 drug product is provided in a liquid dosage form for intravenous (IV) infusion. IMC-002 is provided as a liquid formulation in a 10 mL sterile glass vial, which contains the antibody at a concentration of 500 mg/10 mL (50 mg/mL) in a phosphate-buffered solution comprising 20 mM sodium phosphate, 100 mM sodium chloride, 65.9 mM mannitol, and 0.05% polysorbate 80, at pH 5.5. IMC-002 was diluted in 5% dextrose injection and administered to subjects.

For IMC-002, the drug substance (DS) was manufactured by Samsung Biologics (Republic of Korea), and the drug product (DP) was manufactured by Vetter Pharma (United States).

During the first four doses, all subjects were administered an antihistamine and paracetamol (for example, an intravenous injection of 25 to 50 mg diphenhydramine and 500 to 650 mg paracetamol [acetaminophen] or an oral formulation equivalent thereto) approximately 30 to 60 minutes prior to each dose (or in accordance with local standards). The investigator then decided whether to continue administering premedication for the subjects who did not experience infusion-related reactions. The administered drug was modified in accordance with local treatment standards and guidelines.

### 1.3. Clinical Trial Design

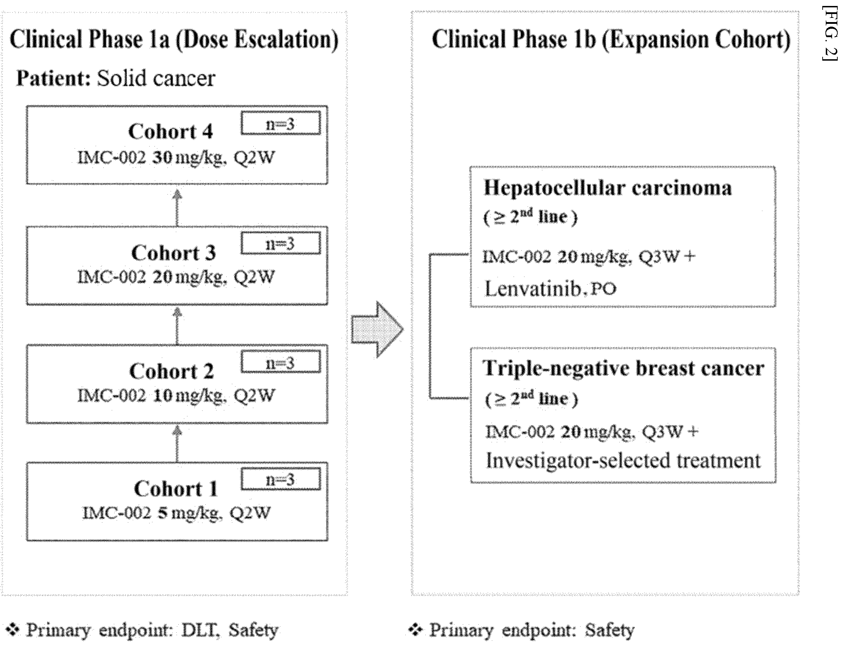

This clinical trial was conducted to evaluate the safety, tolerability, pharmacokinetics, and clinical activity of IMC-002 in patients with advanced cancers who had failed standard therapies. As shown in FIG. 2, the trial consisted two phases: a dose-escalation study (Part 1) and an expansion study (Part 2). In the expansion study, the patients were treated with combination therapy of IMC-002 and other anticancer agents that had not been previously administered.

### 1.3.1. Dose-Escalation Study (Part 1)

Dose-limiting toxicity (DLT) was assessed over a 21-day period using a traditional 3+3 design, and four dose levels of IMC-002 (5, 10, 20, and 30 mg/kg) were administered. IMC-002 was administered intravenously once every 2 weeks for 3 hours ($\pm$30 minutes) (that is, 150 to 210 minutes) until occurrence of progressive disease or unacceptable toxicity. Tumor assessments were performed every six weeks in accordance with RECIST version 1.1 criteria.

Patients with metastatic or locally advanced solid cancers were enrolled who were ineligible for standard therapy or whose cancers were histologically or cytologically confirmed after standard therapy. In addition, the patients were required to have at least one measurable lesion according to the Response Evaluation Criteria in Solid Tumors (RECIST) version 1.1 and an Eastern Cooperative Oncology Group (ECOG) performance status of 0 or 1. RECIST is a well-known set of response evaluation criteria for solid tumors, as described by Eisenhauer et al. (European J. Cancer 45: 228-247, 2009). The Eastern Cooperative Oncology Group (ECOG) performance status scale may be used to assess or select subjects for treatment, for example, those with poor performance following prior therapy (see, for example, Oken et al., Am J Clin Oncol. 5:649-655, 1982). The ECOG performance status scale describes a patient's level of functioning in terms of the patient's ability to care for himself or herself, daily activity, and physical ability (for example, walking, working, and the like).

Twelve refractory patients who met the above-described criteria (9 with hepatocellular carcinoma, 2 with breast cancer, and 1 with gallbladder cancer) were enrolled, with a mean age of 57 years (ranging from 48 to 73 years). Three patients were enrolled at each of dose levels 1 to 3 (5 mg/kg, 10 mg/kg, and 20 mg/kg), and no DLTs were observed. A total of four patients were enrolled at dose level 4 (30 mg/kg), as one patient was deemed ineligible and was not included in the DLT population. The analysis was conducted in the DLT population. Among these patients, 11 had stage IV cancer with multiple distant metastases and a high tumor burden. The median number of prior systemic therapies was three (ranging from 1 to 3), and four patients had received anti-PD-(L)1 antibody treatment. The median interval between the last dose of prior therapy and the administration of IMC-002 was one month (ranging from 1 to 4 months). The baseline characteristics of the enrolled patients are summarized in Table 2 below.

[Table 2]

| Characteristic | 5 mg/kg | 10 mg/kg | 20 mg/kg | 30 mg/kg | Total |
|---|---|---|---|---|---|
| | (n=3) | (n=3) | (n=3) | (n=3) | (N=12) |
| Age, years* | 56 (54-57) | 62 (53-73) | 53 (52-73) | 60 (48-64) | 57 (48-73) |
| Sex, n | | | | | |
|     Male | 3 | 1 | 2 | 2 | 8 |
|     Female | 0 | 2 | 1 | 1 | 4 |
| Race, Asian, n | 3 | 3 | 3 | 3 | 12 |
| ECOG PS 0, n | 2 | 3 | 3 | 3 | 11 |
| Primary disease, n | | | | | |
|     Hepatocellular carcinoma | 3 | 2 | 2 | 2 | 9 |

(continued)

| Characteristic | 5 mg/kg | 10 mg/kg | 20 mg/kg | 30 mg/kg | Total |
| --- | --- | --- | --- | --- | --- |
| | (n=3) | (n=3) | (n=3) | (n=3) | (N=12) |
| Breast cancer | 0 | 1 | 0 | 1 | 2 |
| Gallbladder cancer | 0 | 0 | 1 | 0 | 1 |
| Disease stage IV, n | 3 | 3 | 2 | 3 | 11 |
| Surgical history, n | 3 | 3 | 2 | 3 | 11 |
| RT history, n | 3 | 1 | 1 | 3 | 8 |
| # of prior systemic therapies, n | | | | | |
| 1 | 0 | 2 | 0 | 0 | 2 |
| 2 | 0 | 1 | 1 | 1 | 3 |
| 3 | 3 | 0 | 2 | 2 | 7 |
| Prior anti-PD-(L)1 therapy, n | 2 | 0 | 1 | 1 | 4 |
| Interval between prior therapy and IMC-002 administration, months* | 1.0 (0.7-1.0) | 1.0 (1.0-1.6) | 1.0 (1.0-3.9) | 1.5 (1.3-1.5) | **1.0** (0.7-3.9) |

### 1.3.2. Expansion Study: Combination Administration (Part 2)

Nine patients with hepatocellular carcinoma (HCC) and one patient with triple-negative breast cancer (TNBC) were enrolled as subjects, of whom eight had a history of prior anti-PD-(L)1 therapy. In the expansion study, a combination therapy of IMC-002 with an anticancer agent that the patients had not previously received was used. For the hepatocellular carcinoma cohort, lenvatinib was used in the combination therapy, whereas for the triple-negative breast cancer cohort, gemcitabine + carboplatin was used in the combination therapy.

All subjects received the investigational drug IMC-002 at a dose of 20 mg/kg via intravenous (IV) infusion every 3 weeks (Q3W). During the first cycle, IMC-002 was administered over 3 hours ($\pm$30 minutes). If the first infusion was well tolerated, subsequent infusions were administered over 1 to 1.5 hours ($\pm$10 minutes). The standard of care (SOC; lenvatinib, paclitaxel, or gemcitabine + carboplatin) was initiated after completion of the 1-hour safety monitoring for IMC-002. IMC-002 was administered for up to 2 years (34 cycles) until occurrence of progressive disease (PD) or unacceptable toxicity.

In the hepatocellular carcinoma cohort, lenvatinib was administered once a day at a clinically approved dose of 8 mg (two 4 mg capsules) for subjects weighing less than 60 kg, or 12 mg (three 4 mg capsules) for subjects weighing 60 kg or more. Lenvatinib was administered for up to 2 years (34 cycles) until occurrence of progressive disease or unacceptable toxicity.

For the triple-negative breast cancer cohort, the subjects received gemcitabine + carboplatin for up to 2 years (34 cycles) until occurrence of progressive disease (PD) or unacceptable toxicity. On days 1 and 8 of each cycle, the subjects received gemcitabine at 1,000 $mg/m^2$, followed by intravenous administration of carboplatin at an AUC of 2. The dose of carboplatin was calculated according to the following formula. Gemcitabine was administered over approximately 30 minutes, and carboplatin was administered over approximately 30 to 60 minutes.

$$\text{Dose of carboplatin (mg)} = \text{AUC} \times (\text{GFR} + 25)$$

GFR: Glomerular filtration rate (not normalized to body surface area)

The baseline characteristics of the enrolled patients are summarized in Table 3 below.

[Table 3]

| Characteristic | Total (N=10) |
| --- | --- |
| Age, years* | 61 |
| Sex, n | |
| Male | 9 |

(continued)

| Characteristic | Total (N=10) |
|---|---|
| Female | 1 |
| Race, Asian, n | 10 |
| ECOG PS 0, n | 3 |
| Primary disease, n | |
| Hepatocellular carcinoma | 9 |
| Triple-negative breast cancer | 1 |
| Disease stage IV, n | 9 |
| Surgical history, n | 4 |
| RT history, n | 6 |
| # of prior systemic therapies ≥ 2 | 4 |
| Prior anti-PD-(L)1 therapy, n | 8 |

## 1.4. Safety Results

### 1.4.1. Dose-Escalation Study (Part 1)

No dose-limiting toxicities (DLTs) were observed at any of the four dose levels. The majority of treatment-related adverse events (TRAEs) were Grade 1 or 2 (92%), and most of these events occurred during the first cycle (95%). TRAEs observed in two or more patients included transient vitreous floaters, skin rash, anemia, and nausea. No infusion-related reactions, thrombocytopenia, or neutropenia were reported. The incidence of treatment-related adverse events in the safety population is summarized in Table 4 below.

[Table 4]

| Adverse Event Term | 5 mg/kg (n=3) | 10 mg/kg (n=3) | 20 mg/kg (n=3) | 30 mg/kg (n=3) | Total (N=12) |
|---|---|---|---|---|---|
| Anemia | | | 2 | 3 | 5 (42%) |
| Decreased appetite | | 1 | | | 1 (8%) |
| Diarrhea | | | | 1 | 1 (8%) |
| Fatigue | | | | 1 | 1 (8%) |
| Headache | 1 | 1 | | | 2 (17%) |
| Elevated liver function test values | | | | 1 | 1 (8%) |
| Myalgia | | | 2 | | 2 (17%) |
| Nausea | 1 | | 1 | | 2 (17%) |
| Non-cardiac chest pain | 1 | | | | 1 (8%) |
| Pruritus | | 1 | | | 1 (8%) |
| Pyrexia | | | | 1 | 1 (8%) |
| Skin rash | 2 | 3 | 2 | 2 | 9 (75%) |
| Vitreous floaters | 3 | 3 | | 2 | 8 (67%) |
| Vomiting | | | 1 | | 1 (8%) |

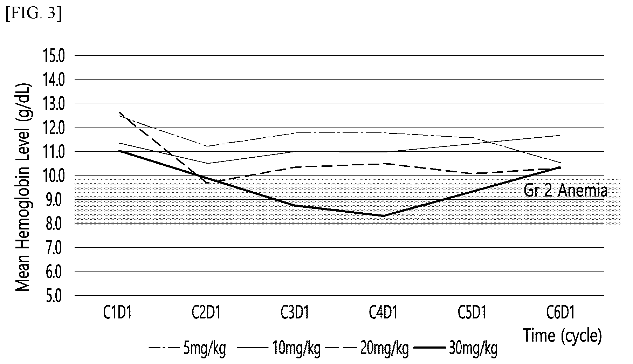

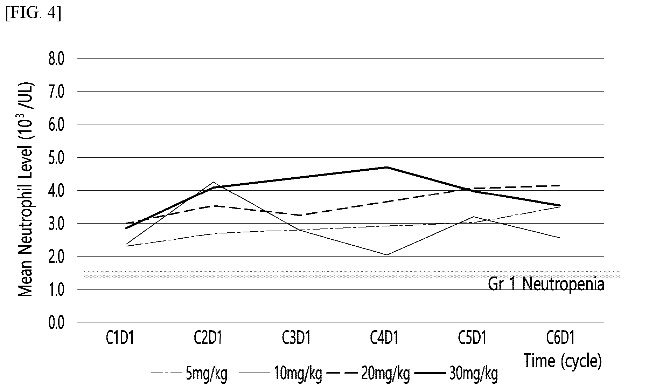

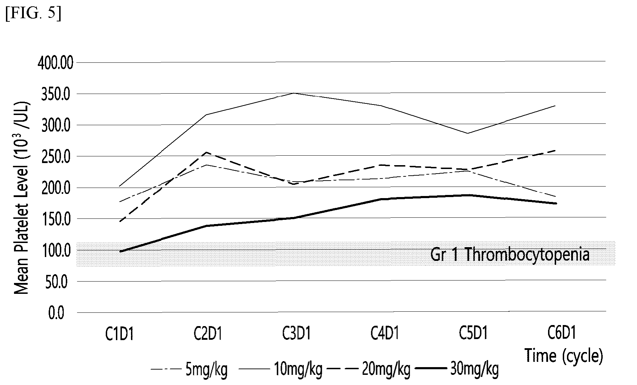

Hemoglobin levels, neutrophil counts, and platelet counts were measured following administration of IMC-002. A slight decrease in hemoglobin levels was observed following initial exposure to IMC-002, and the levels subsequently recovered in the absence of any evidence of hemolytic anemia. Recovery was delayed in the 30 mg/kg cohort compared to the 20 mg/kg cohort. Based on this finding, a dose of 20 mg/kg was selected for administration in the expansion study (Part 2). Neutropenia and thrombocytopenia, which are specific adverse effects seen following initial exposure to anti-CD47 agents, were not observed. Figs. 3 to 5 illustrate that hemoglobin levels, platelet counts, and neutrophil counts remained above the lower limit of normal (LLN) following administration of IMC-002.

### 1.4.2. Expansion Study (Part 2)

A total of 10 subjects were treated with a 3-week combination regimen. As a result, anemia was reported in one subject as a specific adverse effect associated with the anti-CD47 agent. Skin rash and vitreous floaters were observed at a lower incidence compared to the dose-escalation study (Part 1). The incidence of treatment-related adverse events in the safety population is summarized in Table 5 below.

[Table 5]

| Adverse Event Term | 20 mg/kg (N=10) |
|---|---|
| Anemia | 1 (10%) |
| Diarrhea | 1 (10%) |
| Fatigue | 1 (10%) |
| Pyrexia | 1 (10%) |
| Vitreous floaters | 3 (30%) |
| Headache | 1 (10%) |
| Dyspepsia | 1 (10%) |
| Infusion-related reaction | 1 (10%) |
| Skin rash | 4 (40%) |
| Tinnitus | 1 (10%) |
| Elevated thyroid-stimulating hormone | 1 (10%) |

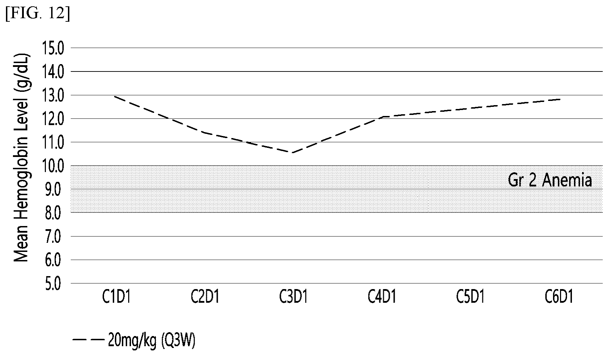

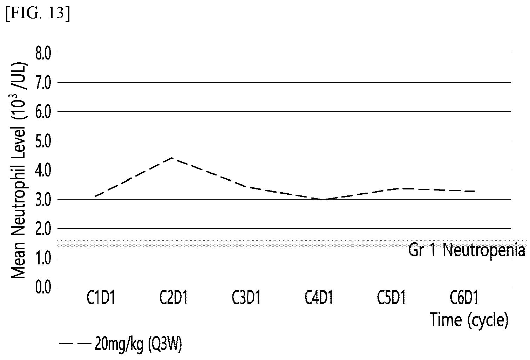

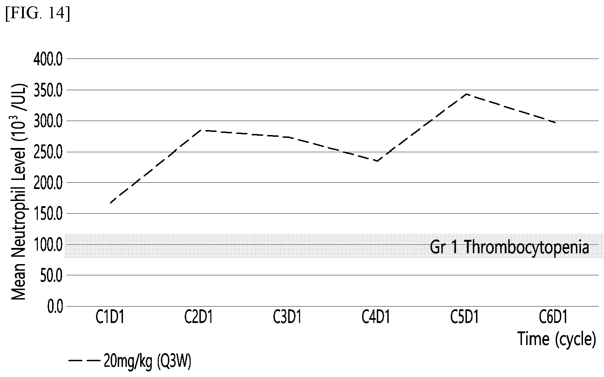

Hemoglobin levels, neutrophil counts, and platelet counts were measured following administration of IMC-002. When IMC-002 was administered according to the 3-week regimen, Grade 2 anemia was not observed. Anemia, neutropenia, and thrombocytopenia, which are specific adverse effects seen following initial exposure to anti-CD47 agents, were not observed. Figs. 12 to 14 illustrate that hemoglobin levels, platelet counts, and neutrophil counts remained above the lower limit of normal (LLN) following administration of IMC-002.

### 1.5. Efficacy Results

### 1.5.1. Dose-Escalation Study (Part 1)

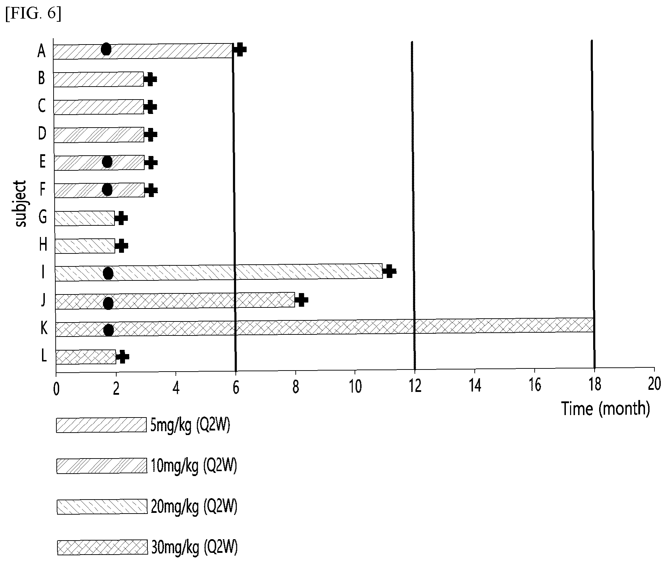

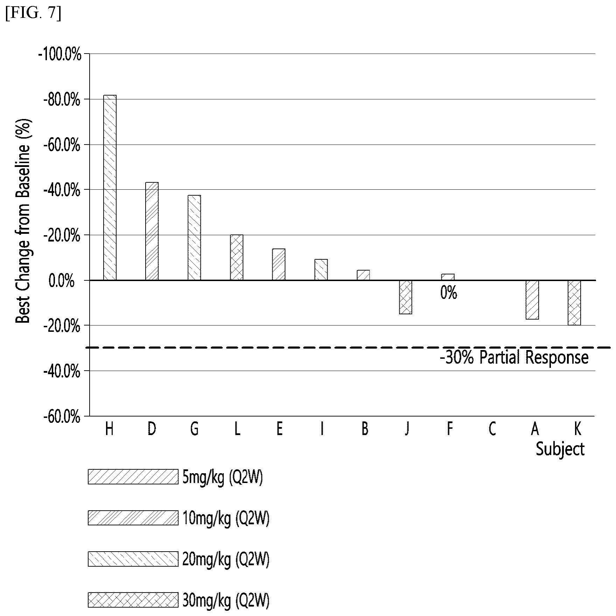

The serum exposure of IMC-002 ($C_{max}$ and AUC) increased in a dose-dependent manner, and the predicted trough concentrations exceeded the minimum effective concentrations of IMC-002 when administered at doses of 10 mg/kg or higher once every 2 weeks. Among the 12 patients evaluable for efficacy, 6 patients exhibited stable disease (disease control rate (DCR) of 50%) with a median treatment duration of 11 cycles (ranging from 6 to 28); and 4 patients maintained stable disease for more than 6 months (clinical benefit rate of 30%). Among these 12 patients, 5 had hepatocellular carcinoma and 1 had breast cancer. Reference is made to Fig. 6, which shows a swimmer plot of tumor responses over time. The percentage change in tumor burden from baseline to the nadir in target lesions is shown in Fig. 7. Referring to the waterfall plot of Fig. 7, a reduction in tumor size (up to - 20%) was observed in 3 out of the 12 patients.

Among the 12 patients evaluable for efficacy, the disease control rate was 50.0%; the clinical benefit rate (CBR, stable disease lasting for at least 6 months) was 33.3%; and the median treatment duration was 10 months. CD47 immunohistochemistry (IHC) images were analyzed using an artificial intelligence (AI)-based analysis tool (Lunit SCOPE) capable of distinguishing staining positivity and cell types at the single-cell level. Specifically, the artificial intelligence (AI)-based analysis tool was used the Lunit SCOPE uIHC v.2 model.

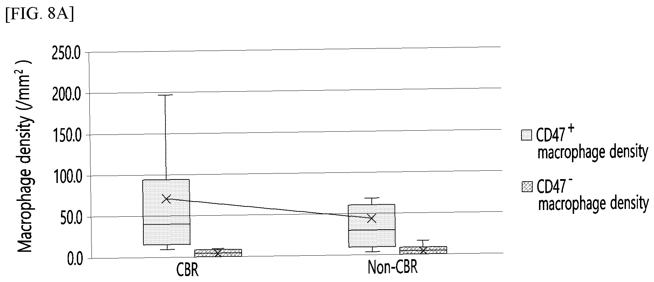

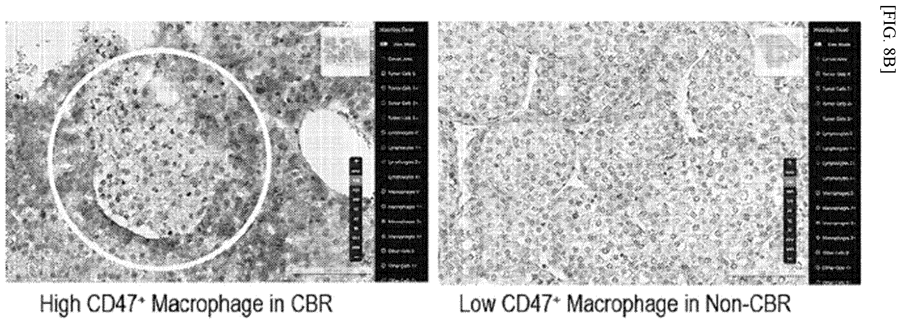

In the artificial intelligence (AI)-based analysis of CD47 IHC, the CD47-positive macrophage density tended to be higher in patients who experienced clinical benefit compared to those who did not (mean CD47[+] macrophage density: 71.0 vs. 44.3 cells/mm$^2$). In contrast, the proportion of CD47-expressing tumor cells was similar between the two groups (mean CD47[+] tumor cell count: 3289.3 vs. 4098.4 cells/mm$^2$). Figs. 8A and 8B illustrate the densities of CD47-positive and CD47-negative macrophages in patients who experienced clinical benefit and those who did not. Higher density of CD47-positive macrophages was observed in the group of patients who experienced clinical benefit. Table 6 below presents the analysis results for the four patients who experienced clinical benefit.

[Table 6]

| Subject (Sex/Age) | Cohort (mg/kg) | Diagnos is | % Change in Target Lesions | Duration (months) | CD47 IHC (%) | CD47+ Tumor Cell Density (cells/mm$^2$) | CD47-Tumor Cell Density (cells/mm$^2$) | CD47+ Macrophage Density (cells/mm$^2$) | CD47- Macrophage Density (cells/mm$^2$) |
|---|---|---|---|---|---|---|---|---|---|
| A (M/56) | 5 | HCC | -17.24 | 6 | 61 | 5876.3 | 3784.0 | 9.2 | 0.4 |
| I (M/73) | 20 | HCC | 9.52 | 11 | 3 | 95.0 | 3339.9 | 17.9 | 8.1 |
| J (M/48) | 30 | HCC | -14.63 | 8 | 84 | 3344.3 | 624.0 | 196.5 | 9.1 |
| K (M/64) | 30 | HCC | -20.00 | 18 | 70 | 3841.7 | 1627.7 | 60.4 | 1.2 |

## 1.5.2. Dose Determination via Pharmacokinetic (PK) Modeling

Population pharmacokinetic (PK) analysis was performed using NONMEM (version 7.5, ICON Development Solution, Ellicott City, MD, USA), supported by PsN (version 3.0.0, Perl-speaks-NONMEM, Uppsala, Sweden). For model development, a total of 213 unbound IMC-002 serum concentration samples from patients were analyzed. Parameter estimation was performed using the first-order conditional estimation with interaction (FOCE-I) method. Model selection was made based on both numerical criteria (for example, a statistically significant decrease in the objective function value [OFV]) and graphical criteria (for example, goodness-of-fit [GOF] plots and visual predictive checks [VPC], n=1000). For nonparametric model evaluation, a bootstrap (n=1000) was performed. Structural models, including general compartment models (for example, one-, two-, and three-compartment models) and a targetmediated drug disposition (TMDD) model incorporating neonatal Fc receptor (FcRn)-mediated recycling, were explored. Stepwise covariate modeling (SCM) was performed to identify clinically relevant covariates. Simulation studies of the final model were conducted under various scenarios to optimize doses for clinical trials.

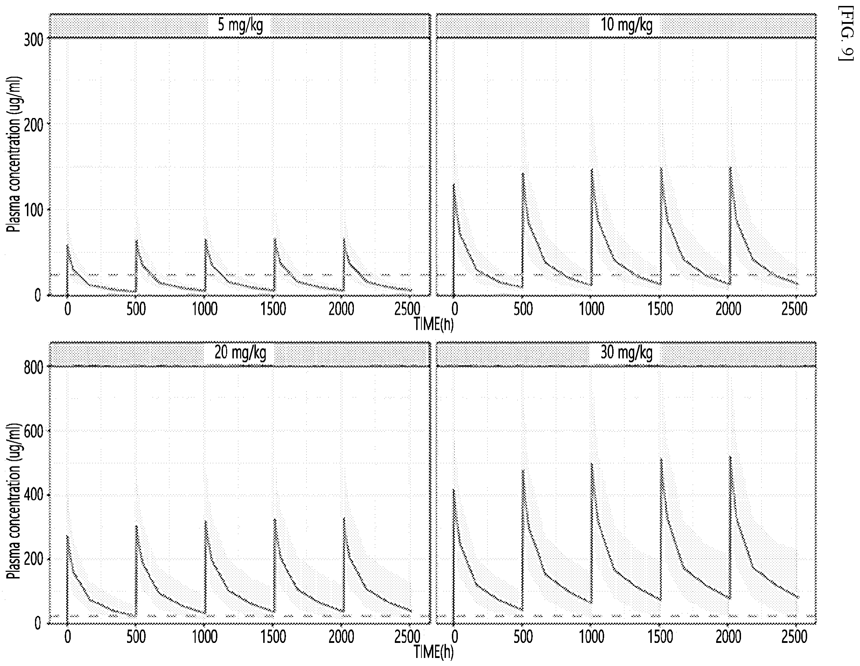

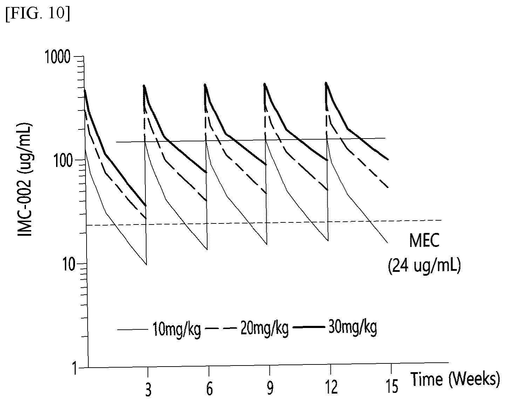

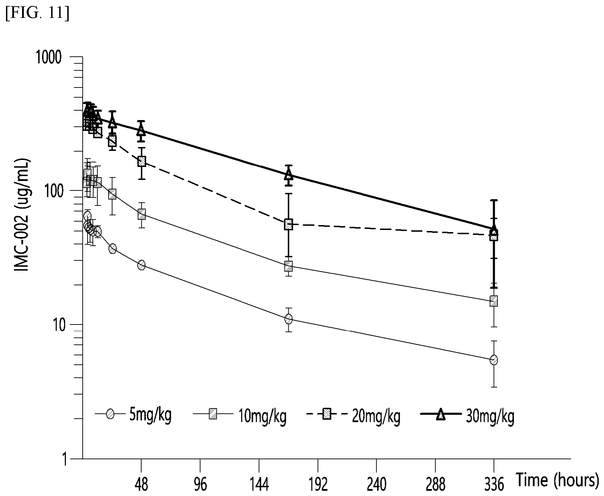

Based on the numerical and graphical criteria, the TMDD model incorporating FcRnmediated recycling of IgG was selected as the final model, as it was able to appropriately describe the pharmacokinetics of IMC-002. In addition, physiological model parameters (for example., degradation rate of CD47 and total receptor amount of FcRn) and binding affinities to targets (for example, binding affinity to CD47, binding affinity to FcRn, and dissociation rate of the drug-CD47 complex) were fixed in the final model based on values obtained from the literature and in vitro observations. No covariate effects on PK of IMC-002 were identified. Simulations were performed using the final model to determine an appropriate dose, which takes into account the minimum effective concentration (MEC, 24 $\mu$g/mL) obtained from the nonclinical efficacy study. As a result, the 3-week interval dosing regimen (Q3W) was considered to be an equally effective regimen compared to the 2-week interval dosing regimen (Q2W) employed in the previous clinical trial. Key parameters at steady state for evaluating whether the regimen is an equally effective regimen are presented in Table 7 and Fig. 9. In Table 7, $AUC_{tau}$ refers to the area under the serum concentration-time curve over the dosing interval, MEC refers to the minimum effective concentration, and $C_{trough}$ refers to the minimum serum concentration of the drug. Fig. 9 illustrates the simulated pharmacokinetic (PK) profile of IMC-002 following intravenous (IV) administration every 3 weeks (Q3W). The solid line represents the median PK profile; the shaded area represents the 5th to 95th percentile range of PK profiles; and the dashed line represents the minimum effective concentration (MEC, 24 $\mu$g/mL). Fig. 10 illustrates a comparison of PK profiles following intravenous administration every 3 weeks at different dose levels. Fig. 11 shows the serum PK concentrations after the initial administration at the different dose levels. Table 7 presents the simulated PK results for the 3-week dosing regimen at cycle 5, at which steady state was confirmed.

[Table 7]

| Parameter | 5 mg/kg | 10 mg/kg | 20 mg/kg | 30 mg/kg |
|---|---|---|---|---|
| $AUC_{tau}$ ($\mu$gh/ml) | 9381 ± 3123 | 24035 ± 11828 | 61847 ± 32098 | 103366 ± 46690 |
| $AUC_{tau}$/MEC ($\mu$gh/ml) | 2349 ± 1713 | 12993 ± 11010 | 49465 ± 32147 | 90984 ± 46827 |
| $C_{trough}$ ($\mu$g/mL) | 6.1 ± 2.3 | 17.1 + 11.8 | 55.9 ± 46.2 | 109.2 ± 74.3 |
| $C_{trough}$ over MEC (%) | - | 14 | 80 | 99 |

IMC-002 is an engineered, fully human IgG4 monoclonal antibody. IgG has a prolonged half-life compared to other immunoglobulins due to the recycling process mediated by the neonatal Fc receptor (FcRn) in the blood and lymphatic circulation. Accordingly, IMC-002 was observed to exhibit similar characteristics to endogenous IgG. This phenomenon is attributed to the nonlinear PK behavior of the drug, which is why the mechanism-based TMDD model was selected as the final model. As IMC-002 was administered based on body weight and all tested covariates (for example, body weight, age, and sex) could be correlated with body weight, it was anticipated that no statistically significant covariates would be identified. Based on the simulation results, considering the duration, for which the plasma concentration remained above MEC observed in the nonclinical and clinical studies, and the maximum tolerated dose (MTD), the 20 mg/kg Q3W regimen was proposed as an effective and safe equivalent dosing regimen. In addition, the half-life of IMC-002 was estimated to be approximately 4 to 5 days, which offers the advantage of being able to synchronize its dosing frequency with other anticancer agents in clinical settings. Since chemotherapy is generally scheduled in 3-week cycles, the drug with a half-life of approximately 4 to 5 days is expected to maintain consistent systemic exposure without accumulation.

### 1.5.3. Expansion Study (Part 2)

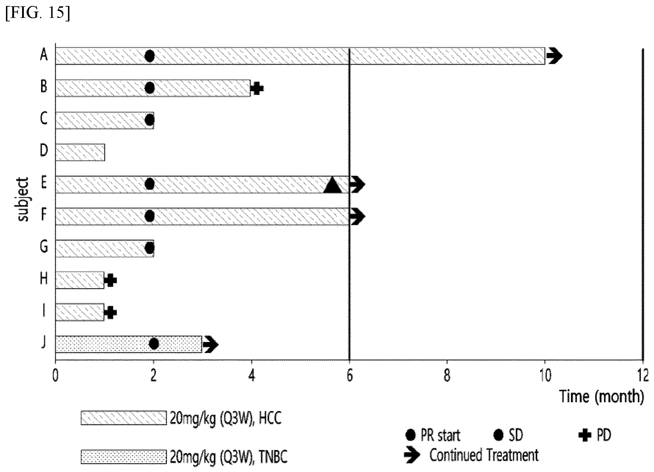

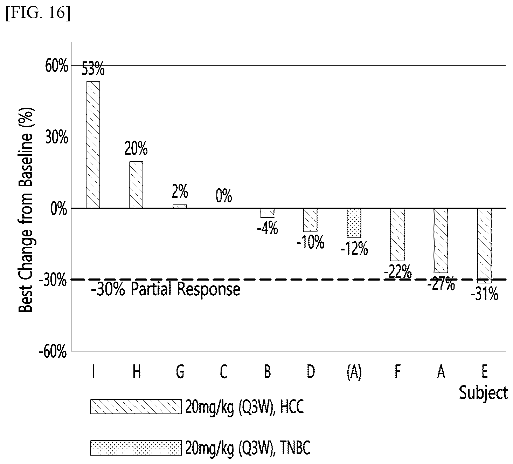

When IMC-002 was administered (in combination administration) at a dose of 20 mg/kg once every 3 weeks (Q3W), 1 of 9 patients with hepatocellular carcinoma exhibited partial response (PR), and 5 patients showed stable disease (SD), resulting in a disease control rate (DCR) of 67%. Among them, 3 patients maintained partial response or stable disease for more than 6 months, resulting in a clinical benefit rate of 33%. Reference is made to Fig. 15 for the swimmer plot showing tumor responses over time. Fig. 16 shows the percentage change in tumor burden from baseline to the nadir in target lesions. Referring to the waterfall plot of Fig. 16, a reduction in tumor size (up to -31%) was observed in 6 out of 10 patients. For the patient with triple-negative breast cancer ((A) of Fig. 16), a 12% reduction in tumor size was also observed.

### Conclusion

In the dose-escalation study, IMC-002 demonstrated an excellent safety profile when administered at doses up to 30 mg/kg every 2 weeks for a treatment duration of up to 18 months, and was found to show preliminary efficacy in the patients with advanced or metastatic solid cancers that had progressed following standard therapy.

Based on the PK modeling and safety data, 20 mg/kg administered every 3 weeks (Q3W) was determined to be an optimal dose, and this dosing regimen was considered to enable more convenient combination administration.

It was found that IMC-002 monotherapy demonstrated a sinificant clinical benefit in the patients with advanced or metastatic solid cancers, in particular, refractory HCC. In addition, it was found that the density of CD47-positive macrophages was higher in the CBR patients than the non-CBR patients.

In the expansion study, it was found that IMC-002, when administered at a dose of 20 mg/kg every 3 weeks, exhibited enhanced safety and clinical activity. Administration of IMC-002 at a dose of 20 mg/kg every 3 weeks showed an excellent safety profile and showed improved clinical benefits when used in combination with other agents such as lenvatinib, paclitaxel, or gemcitabine + carboplatin.

### Claims

1.  A pharmaceutical composition for treating a tumor or inhibiting tumor proliferation in a patient with advanced or metastatic solid cancer, comprising:

    an effective amount of a fully human anti-CD47 antibody that specifically binds to CD47 and comprises HCDR1, HCDR2, and HCDR3 contained in the heavy chain variable region (HCVR) of SEQ ID NO: 1, and LCDR1, LCDR2, and LCDR3 contained in the light chain variable region (LCVR) of SEQ ID NO: 2,
    wherein the antibody is administered to the patient, without a priming dose, at a dose of about 5 mg/kg to about 30 mg/kg about every 2 weeks to about every 3 weeks.

2.  The pharmaceutical composition of claim 1, wherein the heavy chain variable region comprises HCDR1 of SEQ ID NO: 5, HCDR2 of SEQ ID NO: 6, and HCDR3 of SEQ ID NO: 7, and the light chain variable region comprises LCDR1 of SEQ ID NO: 8, LCDR2 of SEQ ID NO: 9, and LCDR3 of SEQ ID NO: 10.

3.  The pharmaceutical composition of claim 1, wherein the heavy chain variable region comprises the amino acid sequence of SEQ ID NO: 1 or an amino acid sequence having at least 95% identity thereto, and the light chain variable region comprises the amino acid sequence of SEQ ID NO: 2 or an amino acid sequence having at least 95% identity thereto.

4.  The pharmaceutical composition of claim 1, wherein the antibody does not induce a significant level of red blood cell agglutination or anemia in the patient.

5.  The pharmaceutical composition of claim 1, wherein the composition comprises about 10 to 30 mM sodium phosphate, about 80 to 120 mM sodium chloride, about 50 to 80 mM mannitol, and about 0.01 to 0.1% polysorbate 80, and has a pH of about 4 to 7.

6.  The pharmaceutical composition of claim 1, wherein the composition comprises about 20 mM sodium phosphate, about 100 mM sodium chloride, about 65.9 mM mannitol, and 0.05% polysorbate 80, and has a pH of about 5.5.

7.  The pharmaceutical composition of claim 1, wherein the composition is in a liquid dosage form for intravenous administration.

8. The pharmaceutical composition of claim 1, wherein the solid cancer is liver cancer, breast cancer, or gallbladder cancer.

9. The pharmaceutical composition of claim 1, wherein the solid cancer is hepatocellular carcinoma.

10. The pharmaceutical composition of claim 1, wherein the solid cancer is triple-negative breast cancer.

11. The pharmaceutical composition of claim 1, wherein the patient is ineligible for standard therapy or has failed standard therapy.

12. The pharmaceutical composition of claim 1, wherein the antibody is administered to the patient about every 2 weeks or about every 3 weeks.

13. The pharmaceutical composition of claim 1, wherein the antibody is administered to the patient at a dose of about 20 mg/kg or about 30 mg/kg.

14. The pharmaceutical composition of claim 1, wherein the patient has received at least one round of systemic therapy before administration of the anti-CD47 antibody.

15. The pharmaceutical composition of claim 1, wherein the patient has stage IV solid cancer with multiple distant metastases or has locally advanced solid cancer.

16. The pharmaceutical composition of claim 1, wherein the antibody is administered in combination with another anticancer agent.

17. The pharmaceutical composition of claim 16, wherein the other anticancer agent is lenvatinib, paclitaxel, gemcitabine, carboplatin, or a combination thereof.

18. The pharmaceutical composition of claim 17, wherein the lenvatinib is administered once a day.

19. The pharmaceutical composition of claim 17, wherein the paclitaxel is administered on day 1 of an administration cycle of the antibody.

20. The pharmaceutical composition of claim 17, wherein the gemcitabine and carboplatin are administered on days 1 and 8 of an administration cycle of the antibody.

21. A method for treating a tumor or inhibiting tumor growth, comprising:

    (a) selecting a patient having advanced or metastatic solid cancer; and
    (b) administering to the patient an anti-CD47 antibody, without a priming dose, at a dose of about 5 mg/kg to about 30 mg/kg about every 2 weeks to about every 3 weeks,

    wherein the antibody specifically binds to CD47 and comprises HCDR1, HCDR2, and HCDR3 contained in the heavy chain variable region of SEQ ID NO: 1, and LCDR1, LCDR2, and LCDR3 contained in the light chain variable region of SEQ ID NO: 2.

22. The method of claim 21, further comprising measuring density of CD47-positive macrophages in a biological sample obtained from the patient before or after administration of the anti-CD47 antibody.

23. The method of claim 21, further comprising:
    (c) additionally administering another anticancer agent before or after administration of the antibody, or concurrently with administration of the antibody.

24. The method of claim 23, wherein the other anticancer agent is administered once a day, or is administered on day 1 of an administration cycle of the antibody, or is administered on days 1 and 8 of an administration cycle of the antibody.

25. A kit for treating a tumor or inhibiting tumor proliferation in a patient with solid cancer, comprising an anti-CD47 antibody together with a manual for using the anti-CD47 antibody,

wherein the manual comprises instructions for administering the antibody to the patient, without a priming dose, at a dose of about 5 mg/kg to about 30 mg/kg about every 2 weeks to about every 3 weeks, and
the antibody specifically binds to CD47 and comprises HCDR1, HCDR2, and HCDR3 contained in the heavy chain variable region of SEQ ID NO: 1, and LCDR1, LCDR2, and LCDR3 contained in the light chain variable region of SEQ ID NO: 2.

[FIG. 1]

[FIG. 2]

**Clinical Phase 1a (Dose Escalation)**

**Patient:** Solid cancer

**Cohort 4** | n=3
IMC-002 **30** mg/kg, Q2W

↑

**Cohort 3** | n=3
IMC-002 **20** mg/kg, Q2W

↑

**Cohort 2** | n=3
IMC-002 **10** mg/kg, Q2W

↑

**Cohort 1** | n=3
IMC-002 **5** mg/kg, Q2W

❖ Primary endpoint: DLT, Safety

**Clinical Phase 1b (Expansion Cohort)**

**Hepatocellular carcinoma**
( ≥ 2nd line )

IMC-002 **20** mg/kg, Q3W +
Lenvatinib, PO

**Triple-negative breast cancer**
( ≥ 2nd line )

IMC-002 **20** mg/kg, Q3W +
Investigator-selected treatment

❖ Primary endpoint: Safety

[FIG. 3]

[FIG. 4]

[FIG. 5]

[FIG. 6]

[FIG. 7]

[FIG. 8A]

[FIG. 8B]

Low CD47+ Macrophage in Non-CBR

High CD47+ Macrophage in CBR

[FIG. 9]

[FIG. 10]

[FIG. 11]

[FIG. 12]

[FIG. 13]

[FIG. 14]

[FIG. 15]

[FIG. 16]

## INTERNATIONAL SEARCH REPORT

| International application No. |
|---|
| **PCT/KR2024/015638** |

**A.    CLASSIFICATION OF SUBJECT MATTER**

**C07K 16/28**(2006.01)i; **A61P 35/00**(2006.01)i; **A61P 35/04**(2006.01)i; **A61K 39/00**(2006.01)i

According to International Patent Classification (IPC) or to both national classification and IPC

**B.    FIELDS SEARCHED**

Minimum documentation searched (classification system followed by classification symbols)

C07K 16/28(2006.01); A61K 39/00(2006.01); A61K 39/395(2006.01); C07K 16/30(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: CD47, 고형암(solid cancer), 항-CD47 항체(anti-CD47 antibody), 프라이밍 (priming)

**C.    DOCUMENTS CONSIDERED TO BE RELEVANT**

| Category* | Citation of document, with indication, where appropriate, of the relevant passages | Relevant to claim No. |
|---|---|---|
| Y | WO 2016-141328 A2 (SORRENTO THERAPEUTICS, INC.) 09 September 2016 (2016-09-09)<br>claims 1, 2, 11, 13-16; page 57, lines 22-26; page 58, lines 26-30; SEQ ID NOs: 8, 22 | 1-20,25 |
| Y | YANG, H. et al. The landscape overview of CD47-based immunotherapy for hematological malignancies. Biomarker Research. (electronic publication) 01 February 2023, vol. 11, thesis no. 15, pp. 1-22.<br>abstract; page 5; table 2 | 1-20,25 |
| A | KR 10-2021-0050538 A (NANJING SANHOME PHARMACEUTICAL CO., LTD.) 07 May 2021 (2021-05-07)<br>claims 1-16 | 1-20,25 |
| A | WO 2020-009725 A1 (TRICAN BIOTECHNOLOGY CO., LTD. et al.) 09 January 2020 (2020-01-09)<br>claims 1-15 | 1-20,25 |
| A | KR 10-2022-0152172 A (IMMUNEONCIA THERAPEUTICS, INC.) 15 November 2022 (2022-11-15)<br>claims 1-3 | 1-20,25 |

☑ Further documents are listed in the continuation of Box C.     ☑ See patent family annex.

| | |
|---|---|
| *    Special categories of cited documents:<br>"A"    document defining the general state of the art which is not considered to be of particular relevance<br>"D"    document cited by the applicant in the international application<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **13 February 2025** | **13 February 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/015638** |

### C.    DOCUMENTS CONSIDERED TO BE RELEVANT

| Category* | Citation of document, with indication, where appropriate, of the relevant passages | Relevant to claim No. |
| --- | --- | --- |
| X | LIM, H. Y. et al. Phase I dose escalation study of IMC-002, a novel anti-CD47 monoclonal antibody, in patients with advanced solid tumors. Annals of Oncology. October 2023, vol. 34, no. S2, 1035P, p. S629. See page S629, left columm. | 1-20,25 |
| X | LIM, H. Y. et al. Phase 1 dose escalation study of IMC-002, a novel anti-CD47 monoclonal antibody, in patients with advanced solid tumors. In: ESMO Congress 2023. poster session 19, 1035P, 21 October 2023. Madrid, Spain. entire document | 1-20,25 |

Form PCT/ISA/210 (second sheet) (July 2022)

## INTERNATIONAL SEARCH REPORT

International application No.

**PCT/KR2024/015638**

**Box No. I**     **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/KR2024/015638** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **21-24**
   because they relate to subject matter not required to be searched by this Authority, namely:

   Claims 21-24 pertain to a method for treatment of the human body by therapy, and thus pertain to a subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
|---|
| **PCT/KR2024/015638** |

| Patent document cited in search report | | | Publication date (day/month/year) | Patent family member(s) | | | Publication date (day/month/year) |
|---|---|---|---|---|---|---|---|
| WO | 2016-141328 | A2 | 09 September 2016 | AU | 2016-225993 | A1 | 28 September 2017 |
| | | | | AU | 2016-225993 | B2 | 24 September 2020 |
| | | | | AU | 2020-294159 | A1 | 28 January 2021 |
| | | | | AU | 2020-294159 | B2 | 02 May 2024 |
| | | | | CA | 2978594 | A1 | 09 September 2016 |
| | | | | CN | 107921121 | A | 17 April 2018 |
| | | | | CN | 107921121 | B | 08 February 2022 |
| | | | | CN | 114380912 | A | 22 April 2022 |
| | | | | CN | 114380912 | B | 23 July 2024 |
| | | | | EP | 3265125 | A2 | 10 January 2018 |
| | | | | JP | 2018-510147 | A | 12 April 2018 |
| | | | | JP | 2020-023532 | A | 13 February 2020 |
| | | | | JP | 2021-054858 | A | 08 April 2021 |
| | | | | JP | 2021-054859 | A | 08 April 2021 |
| | | | | JP | 2021-121635 | A | 26 August 2021 |
| | | | | JP | 6643350 | B2 | 12 February 2020 |
| | | | | JP | 7105938 | B2 | 25 July 2022 |
| | | | | JP | 7137646 | B2 | 14 September 2022 |
| | | | | KR | 10-2018-0006369 | A | 17 January 2018 |
| | | | | KR | 10-2024-0007967 | A | 17 January 2024 |
| | | | | KR | 10-2625835 | B1 | 17 January 2024 |
| | | | | US | 10035855 | B2 | 31 July 2018 |
| | | | | US | 2016-0257751 | A1 | 08 September 2016 |
| | | | | WO | 2016-141328 | A3 | 27 October 2016 |
| KR | 10-2021-0050538 | A | 07 May 2021 | CA | 3110620 | A1 | 05 March 2020 |
| | | | | CA | 3110620 | C | 27 June 2023 |
| | | | | CN | 110872350 | A | 10 March 2020 |
| | | | | CN | 110872350 | B | 07 April 2023 |
| | | | | CN | 112601762 | A | 02 April 2021 |
| | | | | CN | 112601762 | B | 04 April 2023 |
| | | | | EP | 3845561 | A1 | 07 July 2021 |
| | | | | JP | 2021-535743 | A | 23 December 2021 |
| | | | | JP | 2023-075294 | A | 30 May 2023 |
| | | | | JP | 7583707 | B2 | 14 November 2024 |
| | | | | KR | 10-2587442 | B1 | 11 October 2023 |
| | | | | US | 11987627 | B2 | 21 May 2024 |
| | | | | US | 2022-0119520 | A1 | 21 April 2022 |
| | | | | WO | 2020-043188 | A1 | 05 March 2020 |
| WO | 2020-009725 | A1 | 09 January 2020 | CN | 112601544 | A | 02 April 2021 |
| | | | | EP | 3817769 | A1 | 12 May 2021 |
| | | | | JP | 2021-529219 | A | 28 October 2021 |
| | | | | US | 11390677 | B2 | 19 July 2022 |
| | | | | US | 2020-0010546 | A1 | 09 January 2020 |
| KR | 10-2022-0152172 | A | 15 November 2022 | AU | 2022-270543 | A1 | 30 November 2023 |
| | | | | CA | 3219221 | A1 | 10 November 2022 |
| | | | | CN | 117279948 | A | 22 December 2023 |
| | | | | EP | 4335872 | A1 | 13 March 2024 |
| | | | | IL | 308395 | A | 01 January 2024 |
| | | | | JP | 2024-518411 | A | 01 May 2024 |
| | | | | US | 2024-0254233 | A1 | 01 August 2024 |

Form PCT/ISA/210 (patent family annex) (July 2022)

## INTERNATIONAL SEARCH REPORT
### Information on patent family members

| International application No. |
| --- |
| **PCT/KR2024/015638** |

| Patent document cited in search report | Publication date (day/month/year) | Patent family member(s) | Publication date (day/month/year) |
| --- | --- | --- | --- |
| | | WO 2022-235127 A1 | 10 November 2022 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240066776
- KR 102625835

- US 2016020980 W


**Non-patent literature cited in the description**

- **KABAT et al.** Sequences of Proteins of Immunological Interest. US Dept. of Health and Human Services, PHS, NIH, 1991
- **LEFRANC et al.** *Dev. Comp. Immunol.*, 2005, vol. 29, 185-203
- **HONEGGER** ; **PLUCKTHUN**. *J. Mol. Biol.*, 2001, vol. 309 (3), 657-670
- **KOHLER et al.** *Nature*, 1975, vol. 256, 495
- **CLACKSON et al.** *Nature*, 1991, vol. 352, 624-628

- **MARKS et al.** *J. Mol. Biol.*, 1991, vol. 222, 581-597
- **PRESTA**. *J. Allergy Clin. Immunol.*, 2005, vol. 116, 731
- Remington's Pharmaceutical Sciences. 1980
- **EISENHAUER et al.** *European J. Cancer*, 2009, vol. 45, 228-247
- **OKEN et al.** *Am J Clin Oncol.*, 1982, vol. 5, 649-655